# EUROPEAN PATENT SPECIFICATION

(11) **EP 2 153 842 B1**
(45) Date of publication and mention of the grant of the patent: **13.04.2016**
(21) Application number: 09170213.4
(22) Date of filing: 12.10.2006
(51) Int. Cl.: A61K 38/13, A61K 45/06, A61P 27/02

(54) **PREVENTION OF DRUG-ASSOCIATED KERATOCONJUNCTIVITIS WITH A CYCLOSPORIN**
PRÄVENTION VON MIT ARZNEIMITTELN IN ZUSAMMENHANG STEHENDEN KERATOKONJUNCTIVITIS MIT CYCLOSPORIN
PRÉVENTION DE LA KÉRATOCONJONCTIVITE ASSOCIÉE À UN MÉDICAMENT GRÂCE À UNE CYCLOSPORINE

(30) Priority: 14.10.2005 US 596709; 30.11.2005 US 597431 P; 22.06.2006 US 805509 P; 11.10.2006 US 548631
(43) Date of publication of application: 17.02.2010
(62) Divisional of application: 06816746.9
(73) Proprietor: ALLERGAN, INC., Irvine, CA 92612 (US)
(72) Inventor: Schiffman, Rhett M., Laguna Beach, CA 92651 (US); Barnett, Pamela S., Aliso Viejo, CA 92656 (US); Feinermann, Gregg, Irvine, CA 92604 (US); Barth, Neil, Newport Beach, CA 92660 (US)
(74) Representative: Hoffmann Eitle

(56) References cited:
- US-A- 4 839 342
- US-A- 5 294 604
- US-A- 5 411 952
- US-A- 5 977 067
- US-A1- 2002 045 601
- US-A1- 2004 092 435
- US-A1- 2005 025 810
- US-A1- 2005 059 583
- US-B1- 6 245 805
- LANZETTA P ET AL: "Major ocular complications after organ transplantation" TRANSPLANTATION PROCEEDINGS, ORLANDO, FL, US, vol. 36, no. 10, 1 December 2004 (2004-12-01), pages 3046-3048, XP004730809 ISSN: 0041-1345
- LANZETTA P ET AL: "Major ocular complications after organ transplantation", TRANSPLANTATION PROCEEDINGS, ELSEVIER INC, ORLANDO, FL; US, vol. 36, no. 10, 1 December 2004 (2004-12-01), pages 3046-3048, XP004730809, ISSN: 0041-1345, DOI: 10.1016/J.TRANSPROCEED.2004.10.063

## Description

### CROSS-REFERENCE TO RELATED APPLICATIONS

This application claims priority to: United States Provisional Patent Application No. 60/596,709, filed on October 14, 2005; United States Provisional Patent Application No. 60/597,431 , filed on November 30, 2005; and United States Provisional Patent Application No. 60/805,509, filed on June 22, 2006.

### DESCRIPTION OF THE INVENTION

Patients undergoing treatment with certain therapeutically active agents can have certain ocular conditions as a result of that treatment. In particular, patients undergoing chemotherapy with a therapeutically active agent effective for treatment of a cancer often have ocular conditions as a result of that treatment.

The invention is set forth in the claims.

"Administration of a therapeutically active agent to said mammal" means administration of the therapeutically active agent to the mammal in any way that a therapeutically active agent may be administered. Thus, administration of the therapeutically active agent is not limited to the eye, but may include systemic administration via oral, intravenous, rectal, or other means; or administration locally to any part of the body by injection, implantation, topical administration, or other means.

Administration of the therapeutically active agent need not exactly overlap in time with the administration of the cyclosporin or derivative thereof, or a combination thereof. For example, the cyclosporin or derivative thereof, or a combination thereof might be administered to a mammal before the mammal receives any of the therapeutically active agent to avoid the onset of the ocular condition. In another example, the cyclosporin or derivative thereof, or a combination thereof, might be administered after the mammal has begun to receive the therapeutically active agent. In another example, the cyclosporin or derivative thereof, or a combination thereof, might be administered after the mammal has ceased receiving the therapeutically active agent. Administration of the cyclosporin or derivative thereof, or a combination thereof might also be simultaneous with the administration of the therapeutically active agent. Thus, any time relationship may exist between the mammal receiving the therapeutically active agent and the cyclosporin or derivative thereof, or a combination thereof, provided that the use of the latter is reasonably related to treatment or prophylaxis of a condition associated with the former.

It may be convenient to provide a single pharmaceutical composition which comprises both (i) the cyclosporin or derivative thereof, or a combination thereof and (ii) the therapeutically active agent when the agents are to be administered simultaneously.

It may be convenient to provide (i) the cyclosporin or derivative thereof, or a combination thereof and (ii) the therapeutically active agent in form of a kit. For example, the agents may be packaged together. For example, (i) the cyclosporin or derivative thereof, or a combination thereof and (ii) the therapeutically active agent may each be packaged in conventional pharmaceutical packaging such as boxes, jars, blister packs, vials, bottles, syringes etc., and the individually packaged components may then be combined to form a kit e.g. by the use of further packaging such as a box, or by joining up the individual packages. When in kit form, the agents can be taken independently of one another, thus allowing the user freedom to decide the temporal relationship between his use of each of the agents.

Use of a cyclosporin or derivative thereof, including cyclosporin A, for the prophylaxis of keratoconjunctivitis in a person undergoing treatment with a therapeutically active agent for the treatment of cancer is contemplated.

Also contemplated is use of a cyclosporin or derivative thereof, including cyclosporin A, for the prophylaxis of keratoconjunctivitis in a person who is undergoing treatment with an antiviral agent.

Also contemplated is use of a cyclosporin or derivative thereof, including cyclosporin A, for the prophylaxis of keratoconjunctivitis in a person who is undergoing treatment with an immunomodulator.

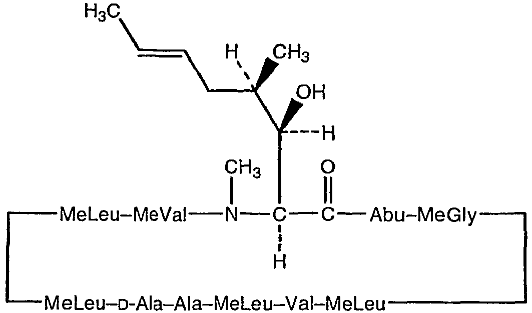

### Cyclosporin A

Cyclosporin A is a cyclic peptide with immunosuppressive properties having the structure shown above. It is also known by other names including cyclosporine, cyclosporine A, ciclosporin, and ciclosporin A.

Other cyclosporins include cyclosporine b, cyclosporine D, cyclosporine G, which are well known in the art. Cyclosporin derivatives and analogs are also known in the art. For example, United States Patent Nos. 6,254,860 and 6,350,442, illustrate several examples. The ocular condition to be prevented is keratoconjunctivitis.

Although the ocular condition may be associated with any antiviral agent, the following antiviral agents are contemplated in particular: Zalcitabine, and Rimantadine Hydrochloride.

While not intending to limit the scope of the invention in any way, the following therapeutically active agents may cause keratoconjunctivitis: Capecitabine.

The therapeutically active agent is administered in the usual manner known in the art for the condition being treated.

Alternatively, a therapeutically active agent and cyclosporin A may be administered in a single composition.

Useful compositions are disclosed in the following patent applications: United States Patent Application Serial No. 11/181, 409, filed on July 1 3, 2005; United States Patent Application Serial No. 11/181, 509, filed on July 1 3, 2005; United States Patent Application Serial No. 11/181, 1 87, filed on July 1 3, 2005; United States Patent Application Serial No. 11/181, 178, filed on July 1 3, 2005; United States Patent Application Serial No. 11/181, 428, filed on July 1 3, 2005; United States Patent Application Serial No. 11/255,821, filed on October 1 9, 2005; United States Patent Application Serial No. 11/161 ,21 8, filed on July 27, 2005; and United States Provisional Patent Application Serial Number 60/727,684, filed on October 1 7, 2005.

In one embodiment, cyclosporin A is administered in the form of Restasis^{®}, available from Allergan, Inc. The cyclosporin A is administered twice a day as indicated on the package insert.

Although there has been hereinabove described pharmaceutical compositions for the purpose of illustrating the manner in which the invention may be used to advantage, it should be appreciated that the invention is not limited thereto. Accordingly, any and all modifications, variations, or equivalent arrangements, which may occur to those skilled in the art, should be considered to be within the scope of the present invention as defined in the appended claims.

## Claims

1. A cyclosporin or derivative thereof, or a combination thereof, for use in combination with a therapeutically active agent, said therapeutically active agent being a chemotherapy agent, an antiviral agent or an immunomodulator, in a method of prophylactically treating keratoconjunctivitis associated with the use of said therapeutically active agent.

2. The cyclosporin for use according to claim 1, wherein said cyclosporin is cyclosporin A.

## Patentansprüche

1. Cyclosporin oder ein Derivat davon oder eine Kombination davon zur Verwendung in Kombination mit einem therapeutischen Wirkstoff, wobei der therapeutische Wirkstoff ein Chemotherapeutikum, ein antivirales Mittel oder ein Immunmodulator ist, in einem Verfahren zur prophylaktischen Behandlung von Keratokonjunktivitis unter Verwendung des therapeutischen Wirkstoffs.

2. Cyclosporin zur Verwendung gemäss Anspruch 1, wobei das Cyclosporin Cyclosporin A ist.

## Revendications

1. Cyclosporine ou dérivé de celle-ci, ou combinaison de celle-ci, pour une utilisation en combinaison avec un agent actif sur le plan thérapeutique, ledit agent actif sur le plan thérapeutique étant un agent de chimiothérapie, un agent antiviral ou un immunomodulateur, dans un procédé de traitement prophylactique de la kératoconjonctivite associée à l'utilisation dudit agent actif sur le plan thérapeutique.

2. Cyclosporine pour une utilisation selon la revendication 1, dans laquelle ladite cyclosporine est la cyclosporine A.
